# EUROPEAN PATENT APPLICATION

(11) **EP 1 249 444 A1**
(43) Date of publication of application: **16.10.2002**
(21) Application number: 01201367.8
(22) Date of filing: 13.04.2001
(51) Int. Cl.: C07C 67/347, C07C 45/50, C07C 69/716

(54) **Continuous hydroformylation process for forming an aldehyde**

(71) Applicant: DSM N.V., 6411 TE Heerlen (NL)
(72) Inventor: Sielcken, Otto Erik, 6136 BG Sittard (NL); Smits, Hubertus Adrianus, 6212 GC Maastricht (NL); Toth, Imre, 6166 GM Geleen (NL)

(57) **Abstract**

A continuous hydroformylation process for forming an aldehyde comprising reacting,
A) an olefinically unsaturated compound;
B) carbon monoxide;
C) hydrogen; and
D) a rhodium-organobisphosphite complex catalyst;
to form a reaction medium wherein the said process is conducted in the presence of between 10 and 800 ppm of an acid compound having a pKa between 1 and 12 measured in water at 18 °C.

## Description

The invention relates to a continuous hydroformylation process for forming an aldehyde comprising reacting,
A) an olefinically unsaturated compound;
B) carbon monoxide;
C) hydrogen; and
D) a rhodium-organobisphosphite complex catalyst;
to form a reaction medium.

It is well known in the art organophosphites may be employed as catalyst ligands for rhodium based hydroformylation catalysts and that such catalysts exhibit exceptional activity and regioselectivity for producing aldehydes via olefin hydroformylation. For instance, U.S. Pat. Nos. 4,668,651 and 4,769,498 describe such hydroformylation processes.

However, despite the benefits attendant with such rhodium-organophosphite complex catalyzed hydroformylation processes, stability of the ligand and catalyst remains a primary concern. The major problem surrounding rhodium-organophosphite catalysts in hydroformylation processes is the inevitable decomposition of the catalysts or phosphite ligands.

The consequences of this problem have been observed during continuous rhodium organophosphite catalyzed hydroformylation reactions. Over time, it has been found that specific acids may form due to degradation of the phosphite ligand in the process. It is believed, without being bound to any theory, that traces of these specific acids have a detrimental effect on the stability of the phosphite ligand thereby causing a loss of ligand concentration. It is therefore necessary to separate these acidic compounds from the catalyst system to avoid the build up of these acids. Alternatively, such a problem may be controlled by passing the liquid reaction effluent stream of the continuous liquid recycle process, either prior to or more preferably, after separation of the aldehyde product therefrom, through a weakly basic anion exchange resin bed, as disclosed, e.g., in U.S. Pat. Nos. 4,668,651; 4,717,775 and 4,769,498. A similar phenomena has been observed when certain organobisphosphite ligands are employed in such hydroformylation processes, which again results in a loss of catalytic activity over time. Such a loss can occur even in the absence of extrinsic poisons, such as chloride or sulfur compounds.

This loss in catalytic activity observed when such organobisphosphite ligand promoted rhodium catalyst systems are employed is believed primarily, or at least partly, due to the formation of a class of by-products which can best be described as decomposition products of the employed organobisphosphite ligands. For example, the organobisphosphite ligand shown employed in the continuous hydroformylation process of propylene in Example 14 of U.S. Pat. No. 4,769,498 (referred to as a polyphosphite ligand in said Example 14) will in time, over the course of the continuous hydroformylation process experience an intrinsic decrease in catalytic activity because of the formation of n-butyl [1,1'-biphenyl-2,2'-diyl] phosphite. Such types of alkyl [1,1'-biaryl-2,2'-diyl] phosphites can coordinate with the rhodium metal and form complexes that are less reactive than the preferred organobisphosphite ligand promoted rhodium catalysts. In effect, such types of alkyl [1,1'-biaryl-2,2'-diyl] phosphites so derived act as a catalyst poison or inhibitor, thereby lowering the catalyst activity of the preferred organobisphosphite ligand promoted rhodium catalyst system.

The object of the invention is to prevent or at least minimize catalyst deactivation in rhodium-bisphosphite complex catalyzed continuous hydroformylation processes.

This object is achieved in that said process is conducted in the presence of between 10 and 800 ppm of an acid compound having a pKa between 1 and 12 measured in water at 18 °C.

It has been found with the process of the invention that deactivation of the catalyst activity is prevented or at least minimized.

The process of the present invention is performed in the presence of between 10 and 800 ppm of an acid compound having a pKa between 1 and 12 measured in water at 18 °C. It has been found that higher concentrations of the acid compound is disadvantageous in that higher concentrations are likely to increase the rate of ligand degradation and/or the formation of high boiling compounds.

In a preferred embodiment of the present invention the rhodium-bisphosphite complex catalyst comprises a bisphosphite ligand of a formula selected from the group consisting of: 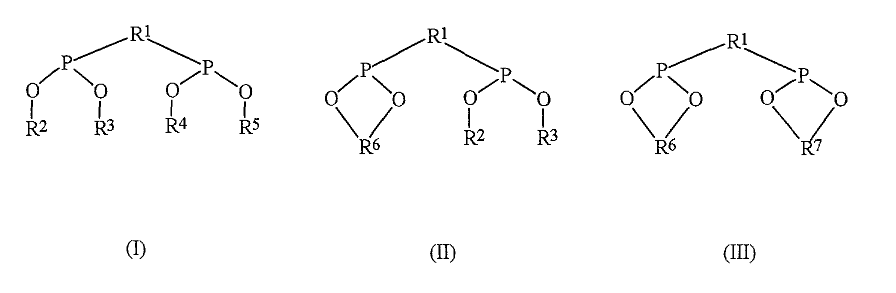 wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different; wherein each Q individually represents a divalent bridging group of -O- or -CR'R"- wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 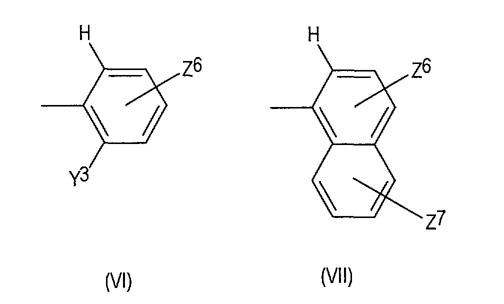 wherein R⁶ and R⁷ might be the same or different and each is individually represented by the structure of (VIII) or (IX), 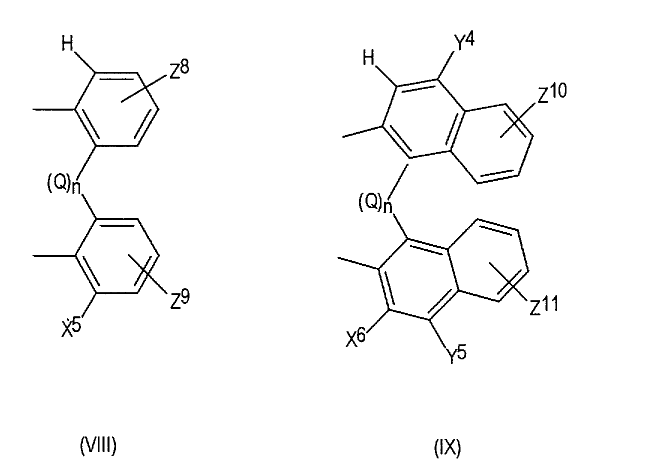 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical, wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

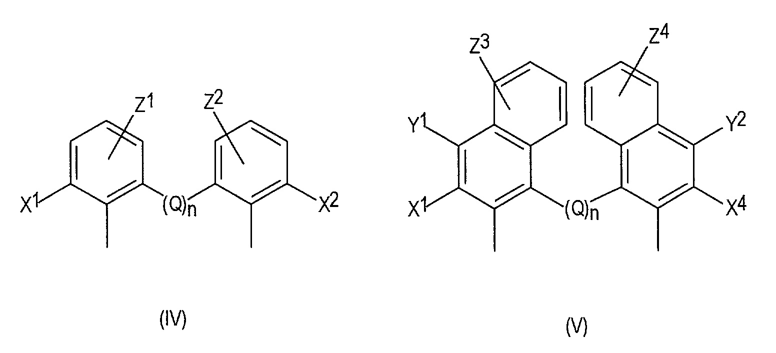

In a more preferred embodiment of the present invention R¹ is represented by the structure of (IV),(V), (VII), (VIIIO, (IX), wherein (Q)ₙ is the same as above,
wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX). Still more preferably said hydroformylation process is performed wherein said ligand used is chosen from the group consisting of [3,3'-bis(t-butyl)-5,5'-dimethoxy-1,1'-biphenyl-2,2'-diyl]-bis(oxy)]-bis(dibenzo[d,f] [1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

The process according to the present invention may add said acid compound in one portion to the reaction medium. Alternately, and more preferably, the process of the present invention may add said acid compound in multiple portions to the reaction medium during the continuous hydroformylation process.

In a preferred embodiment of the present invention, the aldehyde produced is alkyl-5-formylvalerate. Alkyl-5-formylvalerate or a mixture of alkyl-5-, alkyl-4-, alkyl-3- and alkyl-2-formylvalerate is prepared by reaction of alkyl-3-pentenoate or a mixture of alkyl-4-, alkyl-3- and alkyl-2-pentenoate with carbon monoxide and hydrogen in the presence of a hydroformylation catalyst.

In another embodiment of the present invention, monoalkyladipate is generated by reaction of oxygen with alkyl-5-formylvalerate.

Accordingly, the subject invention encompasses preventing or minimizing the intrinsic catalyst deactivation of solubilized rhodium-bisphosphite complex catalyzed, continuous hydroformylation processes for producing aldehydes, by carrying out the hydroformylation in the presence of 10-800 ppm of
said acid compound in reaction medium. More preferably the said acid compound concentration is between 10 and 100 ppm, and still more preferably the said acid compound concentration is between 10 and 50 ppm. The term reaction medium is defined as the liquid solution present within the reaction vessel.

The addition of said acid compound to the reaction medium may be accomplished in a single addition or in periodic or continuous additions over the course of the hydroformylation process. Additionally, the addition of said acid compound may occur during any stage or point of the hydroformylation process, for example, by direct addition to the reaction medium, during the catalyst recycle state, or during the preparation of the system.

Without wishing to be bound to any exact theory or mechanistic discourse, it appears that the structural features of certain bisphosphite ligands which make them such beneficially unique hydroformylation catalyst promoters, as discussed, e.g., in U.S. Pat. No. 4,668,651, are also a cause of the intrinsic catalyst deactivation. For instance while bisphosphite promoted rhodium hydroformylation catalysts of the type employable herein have been found to be highly active and selective in converting terminal as well as internal olefins to aldehydes, it has also been observed that such catalyst systems undergo a loss in catalytic activity over time. It is thought that such loss in catalytic activity is due to the decomposition of the organobisphosphite ligand to acidic phosphorous species. These phosphorous acids, in turn, deactivate the rhodium-organobisphosphite complex, preventing it from catalyzing the desired hydroformylation reaction.

Thus as pointed out herein, a noticeable decrease in the catalytic activity of heretofore conventional continuous rhodium-bisphosphite complex catalyzed continuous hydroformylation processes has been observed to occur over time. This intrinsic loss in catalytic activity manifests itself in terms of a measurable drop in productivity and is considered to be caused by in situ formation of an acidic phosphorous species that poisons the rhodium-bisphosphite complex catalyst, as described herein. Accordingly a basic point of novelty of this invention rests in the discovery that such intrinsic catalyst deactivation in such hydroformylation processes may be prevented or significantly minimized by carrying out the hydroformylation process in the presence of a small amount of said acid compound. It is preferred not to allow for any significant intrinsic catalyst deactivation due to in situ build-up of such monophosphite ligand by-product in the hydroformylation reaction medium, but rather to prevent or at least greatly minimize such deactivation from taking place in the first place by carrying out the hydroformylation process from its start in the presence of 10- 800 ppm of said acid compound.

The olefinic starting material reactants that may be employed in the hydroformylation reactions encompassed by this invention include olefinic compounds containing from 2 to 30 carbon atoms. Such olefinic compounds can be terminally or internally unsaturated and be of straight-chain, branched chain or cyclic structures, as well as be olefin mixtures, such as obtained from the oligomerization of propene, butene, isobutene, etc., (such as so called dimeric, trimeric or tetrameric propylene, and the like, as disclosed, e.g., in U.S. Pat. Nos. 4,518,809 and 4,528,403). Moreover, mixtures of two or more different olefinic compounds may be employed as the starting hydroformylation material if desired. Further such olefinic compounds and the corresponding aldehyde products derived therefrom may also contain one or more groups or substituents which do not unduly adversely affect the hydroformylation process or the process of this invention such as described, e.g., in U.S. Pat. Nos. 3,527,809; 4,668,651 and the like.

Illustrative olefinic unsaturated compounds are alpha-olefins, internal olefins, alkyl alkenoates such as methyl-2-pentenoate, methyl-3-pentenoate and methyl-4-pentenoate, alkenyl alkanoates, alkenyl alkyl ethers, alkenols, and the like, e.g., ethene, propene, 1-butene, 1-pentene, 1-hexene, 1-octene, 1-nonene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 2-butene, 2-methyl propene (isobutylene), 2-methylbutene, 2-pentene, 2-hexene, 3-hexene, 2-heptene, cyclohexene, 2-ethyl-1-hexene, 2-octene, allyl alcohol, allyl butyrate, hex-1-en-4-ol, oct-1-en-4-ol, vinyl acetate, allyl acetate, 3-butenyl acetate, vinyl propionate, allyl propionate, methyl methacrylate, vinyl ethyl ether, vinyl methyl ether, allyl ethyl ether, n-propyl-7-octenoate, 3-butenenitrile, 3-pentenenitrile and 5-hexenamide.

Of course, it is understood that mixtures of different olefinic starting materials can be employed, if desired, by the hydroformylation process of the subject invention. More preferably the subject invention is especially useful for the production of aldehydes, by hydroformylating alpha olefins containing from 2 to 20 carbon atoms, including isobutylene, and internal olefins containing from 4 to 20 carbon atoms as well as starting material mixtures of such alpha olefins and internal olefins. Still more preferably the subject invention is especially useful for the production of aldehydes from methyl-3-pentenonate, in any isomeric form, or mixture of isomeric forms.

The acid can be any acid with a pKₐ between 1 and 12 and preferably between 2.5 and 10, measured in water of 18°C. Examples of suitable acids are aromatic carboxylic acids, for example optionally substituted benzoic acid, p-chloro-benzoic acid, phthalic acid, aliphatic carboxylic acids, for example dicarboxylic acids having between 2-20 carbon atoms, for example adipic acid, glutaric acid and fumaric acid, mono carboxylic acids, for example valeric acid, butynic acid, decanoic acid, is monoalkyladipate, monoalkylmethylglutarate, monoalkylethylsuccinate and/or monoalkylpropylmalonate, phenols, for example phenol, cresol, p-methylphenol, bisphenols, bis-β-naphthol, dihydroxy naphthalene. Preferably the acid has a normal boiling point higher than 200°C.

In case the process of the invention is directed to the preparation of alkyl-5-formylvalerate from alkyl-3-pentenoate or a mixture of alkyl-3-pentenoate with other isomeric forms thereof, preferred acid compounds are monoalkyladipate, monoalkylmethylglutarate, monoalkylethylsuccinate and/or monoalkylpropylmalonate, of which the alkyl group corresponds with the alkyl of the alkyl pentenoate compound.

In one embodiment of the present invention, the desired concentration of monoalkyladipate, monoalkylmethylglutarate, monoalkylethylsuccinate or monoalkylpropylmalonate may be achieved in the reaction mixture by the selective oxidation of alkyl-5-formylvalerate, alkyl-4-formylvalerate, alkyl-3-formylvalerate or alkyl-2-formylvalerate respectively. For example, the desired concentration of monomethyladipate may be achieved in the reaction mixture by the selective oxidation of methyl-5-formylvalerate as shown in equation 1. The presence of molecular oxygen, either in the gas phase or dissolved within the reaction medium, provides a means for a small quantity of methyl-5-formylvalerate to be oxidized to the corresponding acid, monomethyladipate. Thus, an enhancement in catalytic activity is observed in the hydroformylation process when an amount of oxygen gas is present in the reaction mixture in a suitable concentration to oxidize the product aldehyde, for example methyl-5-formylvalerate to monomethyladipate, so that the monomethyladipate concentration is between 10 and 800 ppm, more preferably between 10 and 100 ppm, and still more preferably, between 10 and 50 ppm.

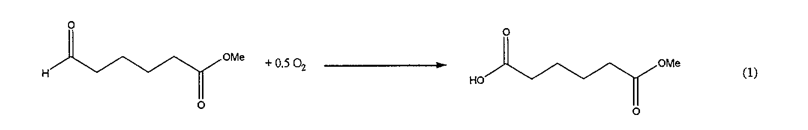

Thus, as described in this embodiment of the invention, oxygen gas and/or dissolved oxygen may provide a means by which monomethyladipate is formed in situ, thus potentially eliminating the need for a separate addition of monomehtyladipate. In addition, oxygen has proven to be a traditional obstacle to rhodium based catalysts employing phosphine or phosphite ligands because of the subsequent decomposition of the catalyst which results from the reaction of oxygen with these reducing species. Therefore, great efforts have been expended to produce industrial reactors which avoid the introduction of air to the reaction mixture. In this embodiment of the present invention, the activity of the rhodium hydroformylation catalyst is actually protected or improved in the presence of a small amount of oxygen and thus affords the great advantage of being able to tolerate some oxygen contamination within the system.
Illustrative rhodium-bisphosphite complex catalysts employable in such hydroformylation reactions encompassed by this invention may include those disclosed in the above mentioned patents wherein the bisphosphite ligand is a ligand selected from the class consisting of Formulas (I) , (II) and (III) above. In general, such catalysts may be preformed, or formed in situ, as described e.g., in said U.S. Pat. Nos. 4,668,651 and 4,769,498, and consist essentially of rhodium in complex combination with the organobisphosphite ligand. It is believed that carbon monoxide is also present and complexed with the rhodium in the active species. The active catalyst species may also contain hydrogen directly bonded to the rhodium.

As noted above illustrative organobisphosphite ligands that may be employed as the bisphosphite ligand complexed to the rhodium catalyst and/or any free bisphosphite ligand (i.e. ligand that is not complexed with the rhodium metal in the active complex catalyst) in such hydroformylation reactions encompassed by this invention include those of Formulas (I), (II), and (III) above.

Illustrative divalent radicals represented by R¹ in the above bisphosphite formulas (I), (II) and (III) include substituted and unsubstituted radicals selected from the group consisting of alkylene, alkylene-(Q)ₙ-alkylene, phenylene, naphthylene, phenylene-(Q)ₙ-phenylene and naphthylene-(Q)n-naphthylene radicals, and where Q, and n are the same as defined above. More specific illustrative divalent radicals represented by R¹ are shown by the structure of (IV) or (V) wherein (Q)ₙ is the same as above. These include, 1,1'biphenyl-2,2'-diyl, 3,3'-dialkyl-1,1'-biphenyl-2,2'-diyl, 3,3'-dicarboxy ester-1,1'-biphenyl-2,2'-diyl, 1,1'binaphthyl-2,2'-diyl, 3,3'-dicarboxy ester-1,1'-binaphthyl-2,2'-diyl, 3,3'-dialkyl-1,1'-binaphthyl-2,2'-diyl, 2,2'-binaphthyl- 1,1'-diyl, phenylene-CH₂-phenylene, phenylene-O-phenylene, phenylene-CH(CH₃)-phenylene radicals, and the like.

Illustrative radicals represented by Z¹, Z², Z³, Z⁴, Z⁵, Z⁶, Z⁷, Z⁸, Z⁹, Z¹⁰, and Z¹¹ in above Formulas (IV) to (IX), in addition to hydrogen, include any of those organic substituents containing from 1 to 18 carbon atoms, disclosed in U.S. Pat. No. 4,668,651, or any other radical that does not unduly adversely effect the process of this invention. Illustrative radicals and substituents encompass alkyl radicals, including primary, secondary and tertiary alkyl radicals such as methyl, ethyl n-propyl, isopropyl, butyl, sec-butyl, t-butyl, neo-pentyl, n-hexyl, amyl, sec-amyl, t-amyl, iso-octyl, decyl, octadecyl, and the like; aryl radicals such as phenyl, naphthyl and the like; aralkyl radicals such as benzyl, phenylethyl, triphenylmethyl, and the like; alkaryl radicals such as tolyl, xylyl, and the like; condensated aryl radicals such as phenylene, naphthylene, and the like, alicyclic radicals such as cyclopentyl, cyclohexyl, 1-methylcyclohexyl, cyclooctyl, cyclohexylethyl, and the like; alkoxy radicals such as methoxy, ethoxy, propoxy, t-butoxy ― OCH₂CH₂OCH₃, ―O(CH₂CH₂)₂OCH₃, ―O(CH₂CH₂)₃OCH₃, and the like; aryloxy radicals such as phenoxy and the like; as well as silyl radicals such as Si(CH₃)₃, ―Si(OCH₃)₃, ―Si(C₃H₇)₃, and the like; amino radicals such as NH₂, ―N(CH₃)₂, ―NHCH₃, ―NH(C₂H₅), and the like; acyl radicals such as C(O)CH₃, ―C(O)C₂H₅, ―C(O)C₆H₅, and the like; carbonyloxy radicals such as ―C(O)OCH₃, ―C(O)OCH(CH₃)₂ ―(C(O)CH(CH₃)C₈H₁₇, and the like; oxycarbonyl radicals such as ―O(CO)C₆H₅, and the like; amido radicals such as ―CONH₂, ―CON(CH₃)₂, ―NHC(O)CH₃, and the like; sulfonyl radicals such as S(O)₂C₂H₅ and the like; sulfinyl radicals such as ―S(O)CH₃ and the like; thionyl radicals such as ―SCH₃, ―SC₂H₅, ―SC₆H₅, and the like; phosphonyl radicals such as ―P(O)(C₆H₅)₂, ―P(O)(CH₃)₂, ―P(O)(C₂H₅)₂, ―P(O)(C₃H₇)₂, P(O)CH₃(C₆H₅), ―P(O)(H)(C₆H₅), and the like.

Illustrative radicals represented by X¹, X², X³, X⁴, X⁵ and X⁶ in above Formulas (IV) to (IX) include those illustrated and discussed above as representing Z¹ to Z¹¹, except hydrogen and condensated aryl radicals.

Illustrative radicals represented by Y¹, Y², Y³, Y⁴ and Y⁵ in above Formulas (IV) to (IX) include those illustrated and discussed above as representing Z¹ to Z¹¹, except condensated aryl radicals.

More preferably, X¹ is the same as X² and Z¹ is the same as Z² in Formula (IV), X³ is the same as X⁴, Z³ is the same as Z⁴, and Y¹, Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

Specific illustrative examples of the bisphosphite ligands employable in this invention include such preferred ligands as:
3,3'-bis(carboxyisopropyl)-1,1'-binaphthyl-2,2'-diyl-bis[bis(1-naphthyl)]phosphite having the formula: 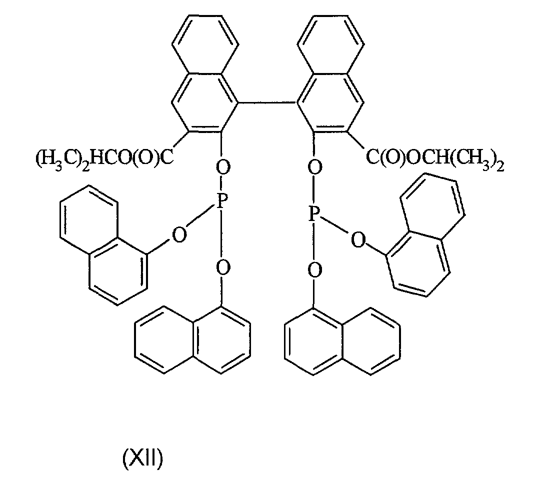
[3,3'-bis(t-butyl)-5, 5'-dimethoxy-1,1'-biphenyl-2,2'-diyl]-bis(oxy)]-bis(dibenzo[d,f] [1,3,2])dioxaphosphepin having the formula: 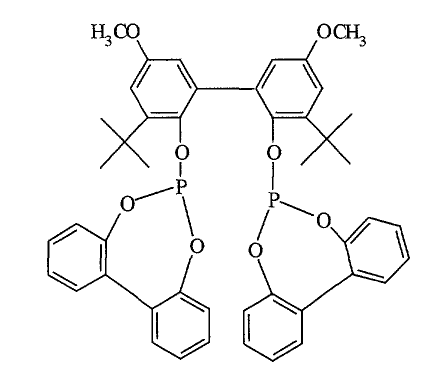
3,3'-bis(carboxyisopropyl)-1,1'-binaphthyl-2-yl-bis[(1-naphthyl)]phosphite-2'-yl-oxy-dibenzo[d,f] [1,3,2]dioxaphosphepin having the formula: 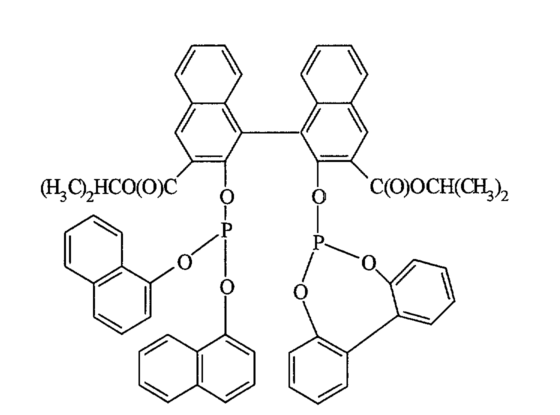
5,5'-bis(t-butyl)-3,3'-dimethoxy-1,1'-biphenyl-2,2'-diyl-bis[bis(1-naphthyl)]phosphite having the formula: 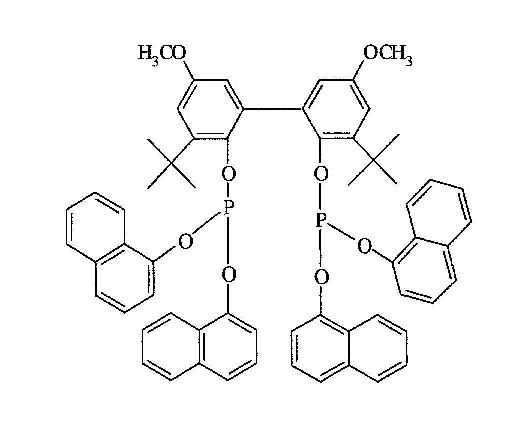
3,3'-bis(carboxymethyl)-1,1'-binaphthyl-2,2'-diyl-bis[bis(2-t-butylphenyl)]phosphite having the formula: 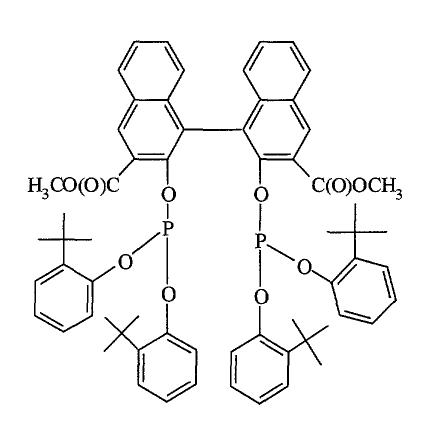
[3,3'-bis(t-butyl)-5,5'-dimethoxy-1,1'-biphenyl-2,2'-diyl]-bis(oxy)]-bis([1,1'-dinaphto[d,f] [1,3,2])dioxaphosphepin having the formula: 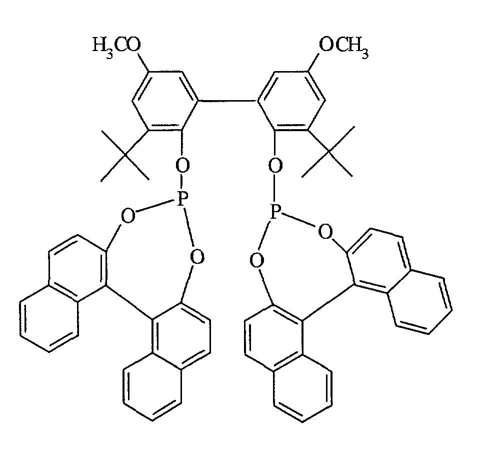
and the like.

Such types of bisphosphite ligands employable in this invention and/or methods for their preparation are well known as seen disclosed for example in U.S. Pat. Nos. 4,668,651; 5,288,918; 5,710,306, the entire disclosure of which is incorporated herein by reference thereto.

In general, such hydroformylation reactions involve the production of aldehydes by reacting an olefinic compound with carbon monoxide and hydrogen in the presence of a rhodium-organobisphosphite complex catalyst in a liquid medium that also contains a solvent for the catalyst. The process may be carried out in a continuous single pass mode or more preferably in a continuous liquid catalyst recycle manner. The recycle procedure generally involves withdrawing a portion of the liquid reaction medium containing the catalyst and aldehyde product from the hydroformylation reaction zone, either continuously or intermittently, and distilling the aldehyde product therefrom in one or more stages, in a separate distillation zone in order to recover the aldehyde product and other volatile materials in vaporous form, the non-volatilized rhodium catalyst containing residue being recycled to the reaction zone. Likewise, the recovered non-volatilized rhodium catalyst containing residue can be recycled with or without further treatment to the hydroformylation zone in any conventional manner desired. Accordingly, the processing techniques of this invention may correspond to any known processing techniques such as heretofore employed in conventional liquid catalyst recycle hydroformylation reactions.

As noted above the hydroformylation reaction conditions that may be employed in the hydroformylation processes encompassed by this invention may include any suitable continuous hydroformylation conditions heretofore disclosed in the above-mentioned patents. Further, the hydroformylation process may be conducted at a reaction temperature from about 25° C. to about 150°. In general hydroformylation reaction temperature of about 70° C. to about 120° are preferred for all types of olefinic starting materials, the more preferred reaction temperatures being from about 90° C. to about 100° C. and most preferably about 95° C.

As noted above the hydroformylation reaction conditions that may be employed in the hydroformylation processes encompassed by this invention may include any suitable continuous hydroformylation conditions heretofore disclosed in the above-mentioned patents. For instance, the total gas pressure of hydrogen, carbon monoxide and olefinic unsaturated starting compound of the hydroformylation process may range from about 1 to about 10,000 psia. In general, however, it is preferred that the process be operated at a total gas pressure of hydrogen, carbon monoxide and olefinic unsaturated starting compound of less than about 1500 psia and more preferably less than about 500 psia. The minimum total pressure being limited predominately by the amount of reactants necessary to obtain a desired rate of reaction. More specifically the carbon monoxide partial pressure of the hydroformylation process of this invention is preferable from about 1 to about 120 psia, and more preferably from about 3 to about 90 psia, while the hydrogen partial pressure is preferably about 15 to about 160 psia and more preferably from about 30 to about 100 psia. In general H₂:CO molar ratio of gaseous hydrogen to carbon monoxide may range from about 1:10 to 100:1 or higher, the more preferred hydrogen to carbon monoxide molar ratio being from about 1:1 to about 10:1. Further, the hydroformylation process may be conducted at a reaction temperature from about 25° C. to about 150°. In general hydroformylation reaction temperature of about 70° C. to about 120° are preferred for all types of olefinic starting materials, the more preferred reaction temperatures being from about 90° C. to about 100° C. and most preferably about 95° C.

As noted above, the continuous hydroformylation process of this invention involved the use of a rhodium-bisphosphite ligand complex catalyst as described herein. Of course mixtures of such catalysts can also be employed if desired. The amount of rhodium-phosphite complex catalyst present in the reaction medium of a given hydroformylation process encompassed by this invention need only be that minimum amount necessary to provide the given rhodium concentration desired to be employed and which will furnish the basis for at least the catalytic amount of rhodium necessary to catalyze the particular hydroformylation process involved such as disclosed e.g. in the above-mentioned patents. In general, rhodium concentrations in the range of from about 10 ppm to about 1000 ppm, calculated as free rhodium, in the hydroformylation reaction medium should be sufficient for most processes, while it is generally preferred to employ from about 10 to 500 ppm of rhodium and more preferably from 25 to 350 ppm to rhodium.

In addition to the rhodium-bisphosphite ligand complex catalyst the hydroformylation process encompassed by this invention may be carried out in the presence of free bisphosphite ligand, i.e. ligand that is not complexed with the rhodium metal of the complex catalyst employed. Said free bisphosphite ligand may correspond to any of the above defined bisphosphite ligands discussed above as employable herein. When employed it is preferred that the free bisphosphite ligand be the same as the bisphosphite ligand of the rhodium-bisphosphite complex catalyst employed. However, such ligands need not be the same in any given process. Moreover, while it may not be absolutely necessary for the hydroformylation process to be carried out in the presence of any such free bisphosphite ligand, the presence of at least some amount of free bisphosphite ligand in the hydroformylation reaction medium is preferred. Thus the hydroformylation process of this invention may be carried out in the absence or presence of any amount of free bisphosphite ligand, e.g. up to 100 moles, or higher per mole of rhodium metal in the hydroformylation reaction medium. Preferably the hydroformylation process of this invention is carried out in the presence of from about 1 to about 50 moles of bisphosphite ligand, and more preferably from about 1 to about 4 moles of bisphosphite ligand, per mole of rhodium metal present in the reaction medium; said amounts of bisphosphite ligand being the sum of both the amount of bisphosphite ligand that is bound (complexed) to the rhodium metal present and the amount of free (non-complexed) bisphosphite ligand present. Of course, if desired, make-up or additional bisphosphite ligand can be supplied to the reaction medium of the hydroformylation process at any time and in any suitable manner, e.g. to maintain a predetermined level of free ligand in the reaction medium.

The hydroformylation reactions encompassed by this invention may also be conducted in the presence of an organic solvent for the rhodium-bisphosphite complex catalyst and any free bisphosphite ligand that might be present. Any suitable solvent which does not unduly adversely interfere with the intended hydroformylation process can be employed. Illustrative suitable solvents for rhodium catalyzed hydroformylation processes include those disclosed e.g. in U.S. Pat. No. 4,668,651. Of course mixtures of one or more different solvents may be employed if desired. Most preferably the solvent will be one in which the olefinic starting material, monomethyladipate, and catalyst, are all substantially soluble. In general, it is preferred to employ aldehyde compounds corresponding to the aldehyde products desired to be produced and/or higher boiling aldehyde liquid condensation by-products as the primary solvent, such as the higher boiling aldehyde liquid condensation by-products that are produced in situ during the hydroformylation process. Indeed, while one may employ any suitable solvent at the start up of a continuous process, the primary solvent will normally eventually comprise both aldehyde products and higher boiling aldehyde liquid condensation by-products due to the nature of such continuous processes. Such aldehyde condensation by-products can also be preformed if desired and used accordingly. Of course, the amount of solvent employed is not critical to the subject invention and need only be that amount sufficient to provide the reaction medium with the particular rhodium concentration desired for a given process. In general, the amount of solvent may range from 0 percent by weight up to about 95 percent by weight or more based on the total weight of the reaction medium.
Moreover as noted herein, the solubilized rhodium-phosphite complex catalyzed continuous hydroformylation process employable in this invention preferably involves a liquid catalyst recycle procedure. Such types of liquid catalyst recycle procedures are known as seen disclosed e.g. in U.S. Pat. Nos. 4,668,651; 4,774,361; 5,102,505 and 5,110,990, and thus need not be particularly detailed herein, since any such conventional catalyst recycle procedures may be employed by this invention. For instance, in such liquid catalyst recycle procedures it is common place to continuously remove a portion of the liquid reaction product medium, containing e.g. the aldehyde product, the solubilized rhodium-bisphosphite complex catalyst, free bisphosphite ligand, and organic solvent, as well as by-products produced in situ by the hydroformylation, e.g. aldehyde condensation by-products etc., and unreacted olefinic starting material, carbon monoxide and hydrogen (syn gas) dissolved in said medium, from the hydroformylation reactor, to a distillation zone, e.g. a vaporizer/separator wherein the desired aldehyde product is distilled in one or more stages under normal, reduced or elevated pressure, as appropriate, and separated from the liquid medium. The vaporized or distilled desired aldehyde product so separated may then be condensed and recovered in any conventional manner as discussed above. The remaining non-volatilized liquid residue which contains rhodium-bisphosphite complex catalyst, solvent, free bisphosphite ligand and usually some undistilled aldehyde product is then recycled back, with or without further treatment as desired, along with whatever by-product and non-volatilized gaseous reactants that might still also be dissolved in said recycled liquid residue, in any conventional manner desired, to the hydroformylation reactor, such as disclosed e.g. in the above-mentioned patents. Moreover the reactant gases so removed by such distillation from the vaporizer may also be recycled back to the reactor if desired.

The distillation and separation of the desired aldehyde product from the rhodium-bisphosphite complex catalyst containing product solution may take place at any suitable temperature desired. In general it is recommended that such distillation take place at low temperatures, such as below 150° C., and more preferably at a temperature in the range of from about 50° C. to about 130° C., and most preferably up to about 115° C. It is also generally recommended that such aldehyde distillation take place under reduced pressure, e.g. a total gas pressure that is substantially lower than the total gas pressure employed during hydroformylation when low boiling aldehydes (e.g. C₄ to C₆) are involved or under vacuum when high boiling aldehydes (e.g. C₇ or greater) are involved. For instance, a common practice is to subject the liquid reaction product medium removed from the hydroformylation reactor to a pressure reduction so as to volatilize a substantial portion of the unreacted gases dissolved in the liquid medium and then pass said volatilized gases and liquid medium which now contains a much lower syn gas concentration than was present in the hydroformylation reaction medium to the distillation zone e.g. vaporizer/separator, wherein the desired aldehyde product is distilled. In general distillation pressures ranging from vacuum pressures or below on up to total gas pressure of about 50 psig should be sufficient for most purposes.

Of course it is to be understood that while the optimization of the subject invention necessary to achieve the best results and efficiency desired are dependent upon one's experience in the utilization of the subject invention, only a certain measure of experimentation should be necessary to ascertain those conditions which are optimum for a given situation and such should be well within the knowledge of one skilled in the art and easily obtainable by following the more preferred aspects of this invention as explained herein and/or by simple routine experimentation.

Finally, the aldehyde products of the hydroformylation process of this invention have a wide range of utility that is well known and documented in the prior art e.g. they are especially useful as starting materials for the production of alcohols and acids, as well as for the production of monomeric and polymeric compounds such as ε-caprolactam, adipic acid, nylon-6 and nylon-6,6.

The following examples are illustrative of the present invention and are not be regarded as limitative. It is to be understood that all of the parts, percentages and proportions referred to herein and in the appended claims are by weight unless otherwise indicated.

### EXAMPLE 1

A solution containing 300 ppm rhodium and a bidentate phosphite ligand with the structure: 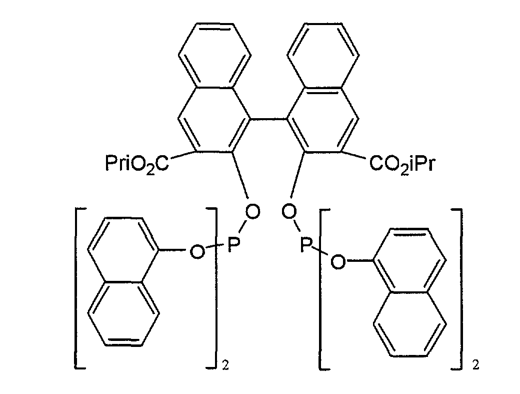 present in a molar ratio to rhodium of 1.1, and tri-ortho-tolyl phosphine in a molar ratio to rhodium of 4 in 600 grams of m-xylene was prepared.

A Hastelloy-B reactor with a nominal volume of 1L was pressurized to 5 bar with an atmosphere of CO and H₂ (the ratio of CO/H₂ =1). The reactor was charged with 200 grams of the above catalyst solution and 300 grams of freshly distilled methyl-3-pentenoate. The mixture was heated to 95°C and continuously stirred, while maintaining the CO/H₂ pressure.

Then a continuous feed was established in which of 90 grams of methyl-3-pentenoate per hour and 80 grams per hour of the above catalyst solution were introduced into the reactor. The catalyst feed was maintained until a total of 475 grams was charged to the reactor. The temperature was maintained at 95°C and the pressure at 5 bar. The level of liquid in the reactor was kept at a maximum of approximately 500 mL of liquid using a dip tube. Via this tube any excess of liquid and unreacted gasses left the reactor. This effluent stream was brought to atmospheric pressure via a back pressure regulator and fed into a gas-liquid separator. This gas was vented and the liquid was collected in the bottom of the separator and fed via a control valve to a first rolled film evaporator operated at 90°C. In this evaporator unreacted methyl-3-pentenoate, light end by-prouducts and a part of the methyl formylvalerate products were removed at a pressure of approximately 0.05 Mpa. The liquid heel of this first evaporator was passed via a column filled with 7 grams of Amberlyst®A-21 ion exchange resin into a second rolled film evaporator. In this evaporator the remainder of the methyl-3-pentenoate, light end by-products, and methylformylvalerate products were evaporated.

The heel of the second evaporator was pumped back into the reactor thereby closing the loop. Approximately 4 hours after starting the methyl-3-pentenoate feed, all evaporators and pumps were operating. After 16 hours the unit reached stable operating conditions. Throughout the run the ligand to rhodium molar ratio was controlled at 1.1 by continuously feeding ligand dissolved in m-xylene to the unit.

It was found that in the first 96 run hours the conversion of the methyl-3-pentenoate dropped gradually from 81% (after 16hours) to 73% after 96 runhours. The selectivity to methyl-5-formylvalerate remained relatively constant at 81.5%.

After 96 hours monomethyladipate was introduced in the unit to a level of 0.01 wt% in the reactor medium, and subsequently maintained at this level by a continuous feed into the reactor. Introduction of the monomethyladipate clearly stopped the deactivation process. It was demonstrated that over the following 100 hours the conversion of the monomethyladipate remained constant at 73%. The selectivity to methyl-5-formylvalerate remained constant at the 81 %-82% level.

This example shows that if a continuous process is performed without the presence of monomethyladipate deactivation of the catalyst occurs. The presence of monomethyladipate stops the deactivation process.

## Claims

1. A continuous hydroformylation process for forming an aldehyde comprising:
reacting,
A) an olefinically unsaturated compound;
B) carbon monoxide;
C) hydrogen; and
D) a rhodium-organobisphosphite complex catalyst;
to form a reaction medium wherein the said process is conducted in the presence of between 10 and 800 ppm of an acid compound having a pKa between 1 and 12 measured in water at 18 °C.

2. Process according to claim 1, wherein the olefinically unsaturated compound is an alkyl-3-pentenoate.

3. Process according to claim 2 wherein the alkyl-3-pentenoate is present in a mixture of alkyl pentenoates having a composition of 0-10% alkyl-2-pentenoate, 0-30% alkyl-4-pentenoate and 60-100% alkyl-3-pentenoate in which the total of alkyl pentenoates add up to 100%.

4. Process according to any one of claims 1-3, wherein the acid compound is monoalkyladipate of which the alkyl group corresponds with the alkyl group of the alkyl pentenoate.

5. Process according to any one of claims 1-3, wherein the acid compound is monoalkyladipate, monoalkylmethylglutarate, monoalkylethylsuccinate and/or monoalkylpropylmalonate, of which the alkyl group corresponds with the alkyl group of the alkyl pentenoate.

6. The process of any one of claims 1-5, wherein the organobisphosphite ligand of said rhodium-organobisphosphite complex catalyst is a ligand selected from the class consisting of

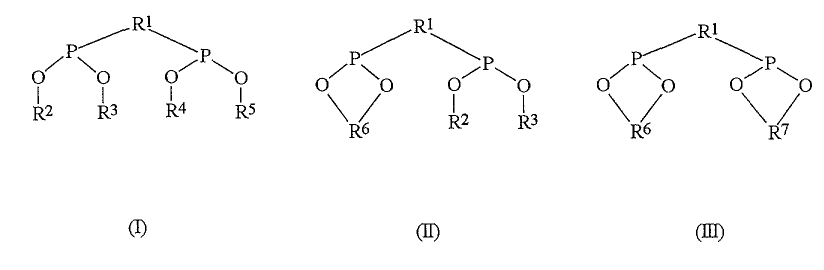

7. wherein each R¹ represents a divalent radical selected from a group consisting of alkylene, alkylene-(Q)ₙ-alkylene, arylene and arylene-(Q)ₙ-arylene, and wherein each alkylene radical individually contains from 2 to 18 carbon atoms and is the same or different, and wherein each arylene radical individually contains from 6 to 18 carbon atoms and is the same or different;
wherein each Q individually represents a divalent bridging group of -O- or -CR'R"- wherein each R' and R" radical individually represents hydrogen or a methyl radical; and wherein each n individually has a value of 0 or 1,
wherein R², R³, R⁴, and R⁵ might be the same or different and each is individually represented by the structure of (VI) or (VII), 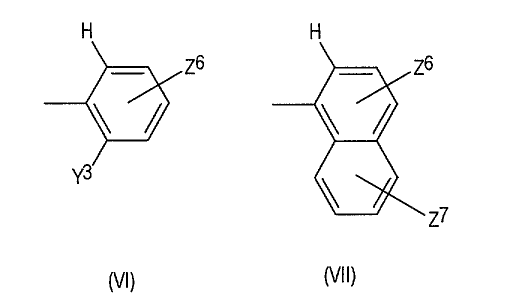 wherein R⁶ and R⁷ might be the same or different and each is individually represented by the structure of (VIII) or (IX), 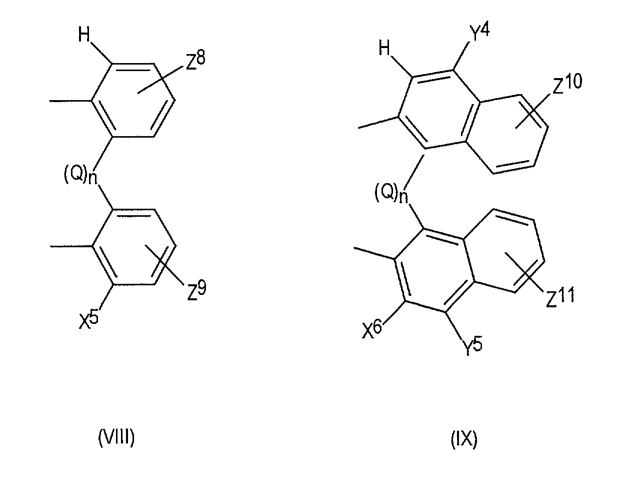 wherein X¹, X², X³, X⁴, X⁵ and X⁶ might be the same or different and each individually represents an organic radical, wherein Y¹, Y², Y³, Y⁴ and Y⁵ are the same or different and each represents a hydrogen or alkyl radical,
wherein Z¹, Z², Z³, Z⁴, Z⁵, Z⁶, Z⁷, Z⁸, Z⁹, Z¹⁰ and Z¹¹ might be the same or different and each represent a hydrogen or an organic radical placed at any remaining position of the aryl rings of structures (IV) to (V),

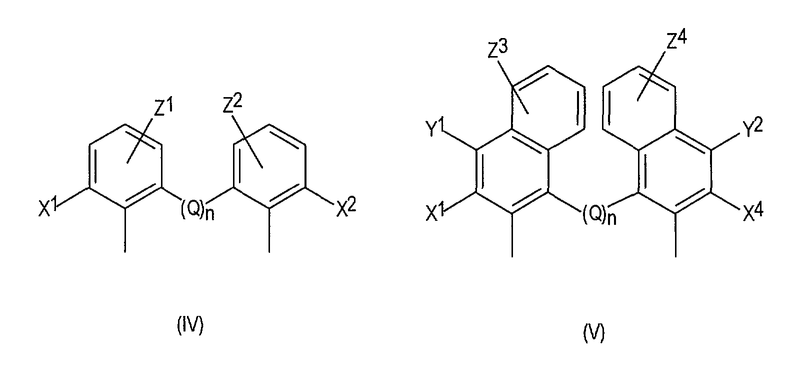

8. The process of claim 6, wherein R¹ is represented by the structure of (IV), (V), (VII), (VIII) or (IX) wherein X¹ is the same as X² and Z¹ is the same as Z² in Formula (IV), X³ is the same as X⁴, Z³ is the same as Z⁴, and Y¹ and Y² are hydrogen radicals in Formula (V), Z⁶ is a hydrogen radical in Formula (VII), Z⁸ is the same as Z⁹ in Formula (VIII), Z¹⁰ is the same as Z¹¹ and Y⁴ and Y⁵ are hydrogen radicals in Formula (IX).

9. The process according to any one of claims 6-7, wherein the ligand used is [3,3'-bis(t-butyl)-5,5'-dimethoxy-1,1'-biphenyl-2,2'-diyl]-bis(oxy)]-bis(dibenzo[d,f][1,3,2])dioxaphosphepin, 3,3'-bis(carboxyisopropyl)-1,1'-binaphthyl-2,2-diyl-bis[bis(1-naphthyl)]phosphite and 3,3'-bis(carboxymethyl)-1,1'-binaphthyl-2,2'-diyl-bis[bis(2,5-di-t-butyl)]phosphite.

10. The process according to any one of claims 1 through 8 wherein the amount of the acid compound present is between 10 and 100 ppm.

11. The process according to claim 9 wherein the amount of acid compound present is between 10 and 50 ppm.

12. The process according to any one of claims 1-5 wherein said acid compound is added in one portion to the reaction medium.

13. The process according to any one of claims 1-5 wherein said acid compound is added in multiple portions to the reaction medium during the continuous hydroformylation process.

14. The process according to any one of claims 1-5, wherein said monoalkyladipate is generated by reaction of oxygen with alkyl-5-formylvalerate.
